# EUROPÄISCHE PATENTSCHRIFT

(11) **EP 2 735 337 B1**
(45) Veröffentlichungstag und Bekanntmachung des Hinweises auf die Patenterteilung: **30.09.2015**
(21) Anmeldenummer: 13193746.8
(22) Anmeldetag: 21.11.2013
(51) Int. Cl.: A61N 5/06, A61H 39/04, A61H 39/00

(54) **Therapiegerät**
Therapy device
Appareil de thérapie

(30) Priorität: 22.11.2012 DE 102012111266
(43) Veröffentlichungstag der Anmeldung: 28.05.2014
(73) Patentinhaber: Jochum, Günther, 66578 Schiffweiler (DE)
(72) Erfinder: Jochum, Günther, 66578 Schiffweiler (DE)
(74) Vertreter: Wolff, Matthias

(56) Entgegenhaltungen:
- DE-A1- 3 911 586
- DE-A1- 4 009 644
- DE-A1- 4 444 756
- DE-A1-102010 060 605

## Beschreibung

Die Erfindung betrifft ein Therapiegerät, das eine Strahlungsquelle, insbesondere eine Lichtquelle, zur Bestrahlung eines Patienten, und einen pyramidenartig geformten Körper, der im Strahlengang des Therapiegeräts angeordnet ist, aufweist.

Aus der DE 44 44 756 A1 ist ein Therapiegerät der eingangs genannten Art bekannt. In dem Therapiegerät ist im Strahlengang hinter einer Strahlungsquelle ein Filter angeordnet, der aus Kunststoff gebildet ist, in dem Edelsteinstücke geringer Korngröße angeordnet sind. Einer der beschriebenen Filter ist einteilig gebildet und weist eine Pyramidenform auf. Ferner ist ein weiterer einteiliger Filter beschrieben, der im Querschnitt eine kreisrunde Form aufweist, mit 32 Facetten versehen ist und an einer Ober- und einer Unterseite, zwischen denen eine Rondiste vorgesehen ist, pyramidenartige Spitzen aufweist.

Weitere durch Benutzung bekannte Therapiegeräte, die eine Strahlungsquelle zur Bestrahlung von Patienten umfassen, werden zur Behandlung verschiedener Krankheiten, insbesondere Hautkrankheiten, verwendet.

Der Erfindung liegt die Aufgabe zugrunde, ein Therapiegerät der eingangs genannten Art zu schaffen, mit dem weitere Therapien möglich werden.

Diese Aufgabe wird dadurch gelöst, dass das Therapiegerät zumindest zwei der pyramidenartig geformten Körper aufweist, die Körper mit ihren Grundflächen unmittelbar oder über eine Zwischenlage aneinander anliegen und im Strahlengang des Therapiegeräts hintereinander angeordnet sind, wobei einer der Körper aus Opal und der andere oder die anderen Körper aus Kristall oder aus einem Materialgerüst einer Koralle gebildet ist bzw. sind.

Zur Behandlung wird mittels des Therapiegeräts die Haut des Patienten mit der von den Körpern ausgehender Strahlung bestrahlt. Insbesondere wenn Akupunkturpunkte und/oder Chakren des Patienten bestrahlt werden, können Leiden gelindert oder sogar vollständig behoben werden. Als besonders wirkungsvoll hat sich das Therapiegerät zur Behandlung von chronischen Krankheiten, insbesondere chronische Hautkrankheiten, chronische Darmerkrankungen, Erkrankungen des rheumatischen Formenkreises, Abwehrschwäche, Fibromyalgie, chronisches Müdigkeitssyndrom, multiple Chemistry-Syndrom, Demenz, Tumorerkrankungen, Kopfschmerzen und Wirbelsäulenschmerzen erwiesen. Es versteht sich, dass die Behandlung mit dem Therapiegerät ergänzend zur klassischen medizinischen Behandlung angewendet werden kann.

Überraschend hat der Erfinder herausgefunden, dass bei Bestrahlung der Körper die Strahlung, insbesondere das Licht, aufgrund der kristalleigenen Energie und Struktur transformiert wird und die Kristalle daraufhin die kristalleigene Energie abgeben. In Analogie zu den Forschungen und Experimenten von Prof. Masaru Emoto, nach denen Information in Wasser gespeichert ist, enthalten auch die Kristalle eine gespeicherte Information. Durch die Behandlung mit dem Therapiegerät werden sowohl die kristalleigenen Schwingungen als auch die Informationen der Kristalle auf den Patienten übertragen. Dies führt zu einer verbesserten Körperregulation und eine verbesserte Zellregeneration wird möglich.

Zweckmäßigerweise strahlt die Strahlungsquelle im Wellenlängenbereich des sichtbaren Lichts. Um eine Hitzeentwicklung bei der Behandlung zu vermeiden, hat es sich als zweckmäßig erwiesen, eine Kaltlichtquelle zu verwenden, bei der der Infrarotanteil der Strahlung gering oder nicht vorhanden ist. Vorzugsweise weist das Licht Wellenlängen < 780 nm auf. Als vorteilhaft hat es sich erwiesen, lediglich blaues Licht deshalb im Wellenlängenbereich von 460 bis 480 nm zu verwenden.

In der bevorzugten Ausführungsform der Erfindung ist die Strahlungsquelle durch eine Leuchtdiode (LED) gebildet.

In der bevorzugten Ausführungsform der Erfindung weisen die Körper die Form einer Pyramide, vorzugsweise einer regelmäßigen Pyramide, auf. Zweckmäßigerweise ist die Grundfläche der Pyramide quadratisch, wobei die Grundfläche und die Seitenflächen vorzugsweise in einem Winkel von 51° 50s zueinander angeordnet sind.

Zweckmäßigerweise ist die Zwischenlage durch ein Plättchen, eine Folie und/oder durch ein Pulver gebildet, wobei die Zwischenlage vorzugsweise die Form eines fünfzackigen Sterns aufweist.

Besonders gute Therapieergebnisse lassen sich erzielen, wenn die Zwischenlage aus Gold gebildet ist.

In einer weiteren Ausgestaltung der Erfindung ist zumindest einer der Körper an der Fläche, mit der er an dem anderen Körper anliegt, mit einer Gravur versehen. Die Gravur kann die Form von Außenkonturen eines, vorzugsweise 5-zackigen, Sterns aufweisen. In der bevorzugten Ausführungsform der Erfindung ist in der Gravur Goldstaub angeordnet.

In einer Ausführungsform der Erfindung sind die Körper und ggf. die Zwischenlage in einer Baueinheit zusammengefasst, die in dem Therapiegerät austauschbar ist. Das Therapiegerät kann mehrere der Baueinheiten umfassen, die jeweils mit Körpern unterschiedlicher Materialkombinationen versehen sind, beispielsweise schwarzer Opal mit Saphir, schwarzer Opal mit Smaragd oder Aquamarin mit rosa Koralle. Zweckmäßigerweise sind die Körper durch zwei, vorzugsweise aus Silber gebildeten und mit einer Ausnehmung zur Aufnahme der Körper versehenen, Scheiben zusammengehalten. Die Scheiben können durch Schweißen miteinander verbunden sein.

In einer weiteren Ausgestaltung der Erfindung ist im Strahlengang hinter den beiden Körpern ein, vorzugsweise aus Bergkristall gebildetes, Kontaktelement vorgesehen, das bei der Behandlung auf die Haut des Patienten auflegbar ist.

Das Kontaktelement, das zweckmäßigerweise eine zylindrische Form aufweist und sich vorzugsweise an dem Ende, mit dem es auf den Patienten aufzusetzen ist, konusartig verjüngt, hat sich insbesondere bei der Bestrahlung der Akupunkturpunkte als für die Behandlung vorteilhaft erwiesen.

In einer weiteren Ausführungsform der Erfindung ist das Kontaktelement konusartig geformt und auf seiner der Strahlungsquelle zugewandten Seite mit einer Auswölbung versehen, in welcher eine Spitze zu dem Strahlengang hinteren Körpers vorstehen kann.

In einer besonders bevorzugten Ausgestaltung der Erfindung weist das Therapiegerät eine durch eine rohrförmige, vorzugsweise aus Silber gebildete, Hülse zur Aufnahme der Strahlungsquelle, der Körper und ggf. des Kontaktelements auf, wobei zweckmäßigerweise eine Einrichtung zur Verstellung des Abstands zwischen der Strahlungsquelle und der Körper vorgesehen ist.

Während es vorstellbar wäre, die Position der Körper in der Hülse verstellbar vorzusehen, ist in einer bevorzugten Ausgestaltung der Erfindung die Position der Strahlungsquelle in der Hülse verstellbar.

Die Erfindung ist in den Ansprüchen definiert. Andere Ausführungsformen sind lediglich beispielhaft.

Die Erfindung wird nachfolgend anhand eines Ausführungsbeispiels und der beigefügten, sich auf dieses Ausführungsbeispiel beziehenden Zeichnungen und Tabellen näher erläutert. Es zeigen:
- Fig. 1: ein erfindungsgemäßes Therapiegerät im Schnitt,
- Fig. 2: ein Bauteil des Therapiegeräts nach Fig. 1 in Draufsicht,
- Fig. 3: das Bauteil nach Fig. 2 in Seitenansicht,
- Fig. 4 bis 6: Details des Bauteils nach den Fig. 2 und 3 in verschiedenen Ansichten,
- Fig. 7: ein erfindungsgemäßes Therapiegerät in perspektivischer Ansicht von der Seite,
- Fig. 8: Bauteile des Therapiegeräts nach Fig. 7,
- Fig. 9: einen Teil des Therapiegeräts nach Fig. 7 im Schnitt, und
- Tab. 1 bis 3: Messergebnisse vor und nach Behandlung von Patienten mit dem Therapiegerät.

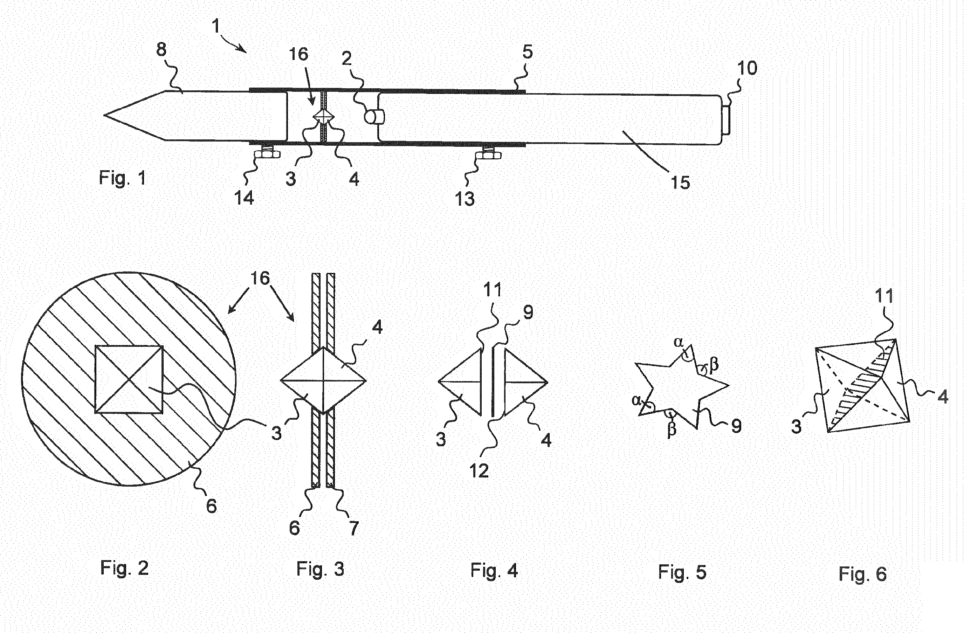

Ein in Fig. 1 gezeigtes erfindungsgemäßes Therapiegerät 1 weist eine Hülse 5 aus Silber auf, in der ein zylindrisches Trägerelement 15 angeordnet ist, an dessen einem Ende eine Leuchtdiode 2 und an dessen anderen Ende ein Schalter 10 zum Ein- und Ausschalten der Leuchtdiode 2 vorgesehen ist. In dem Trägerelement 15 ist ferner eine hier nicht gezeigte Energiequelle für die Leuchtdiode 2 angeordnet. Zur Verstellung des zylindrischen Körpers ist in der Hülse 5 eine Gewindebohrung vorgesehen, in der eine Schraube 13 angeordnet ist, mit der sich die Position des Trägerelements 15 feststellen lässt.

In der Hülse 5 ist neben der Leuchtdiode 2 ein Baueinheit 16 angeordnet, die näher in den Figuren 2 bis 6 gezeigt ist.

Wie sich insbesondere den Figuren 3, 4 und 6 entnehmen lässt, weist die Baueinheit 16 zwei Körper 3,4 auf, die die Form einer Pyramide aufweisen und die mit ihren Grundflächen 11,12 aneinander liegen. Der Körper 4 ist aus schwarzem Opal und der Körper 3 aus Amethyst gebildet.

In den Figuren 2 und 3 ist gezeigt, dass die Körper 3,4 durch zwei aus Silber gebildete Scheiben 6,7 der Baueinheit 16 gehalten sind. Die Scheiben 6,7 sind kreisförmig und mit ihrem Rand an der Innenseite der Hülse 5 befestigt. Sie weisen je eine entsprechend der Form der Körper 3,4 abgeschrägte Ausnehmung auf, in die die Körper 3,4 eingesetzt werden. Durch die Scheiben 6,7 werden die Körper 3,4 aneinander gehalten. Die Körper 3,4 weisen die Form einer regelmäßigen Pyramide mit quadratischer Grundfläche auf, wobei zwischen der jeweiligen Grundfläche und den Seitenflächen ein Winkel von 51° 50s vorgesehen ist.

In einer hier nicht gezeigten Ausführungsform umfasst das Therapiegerät 1 mehrere Baueinheiten 16, die mit Körpern 3,4 unterschiedliche Materialkombinationen besetzt sind, z.B. schwarzer Opal mit rosa Turmalin, schwarzer Opal mit Aquamarin oder Wassermelonenturmalin mit Rubin. Diese Baueinheiten 16 sind in dem Therapiegerät 1 austauschbar. Dazu kann die Hülse 5 mit einem Schlitz versehen sein, durch den hindurch sich die Baueinheiten 14 austauschen lassen. Zweckmäßigerweise ist eine Einrichtung zur Befestigung der Baueinheiten in der Hülse 5 vorgesehen. Der Schlitz ist ggf. verschließbar.

Wie insbesondere Fig. 4 zu entnehmen ist, kann zwischen den Grundflächen 11,12 der Körper 3,4 eine dünne aufgedampfte Schicht 9 angeordnet sein, die, wie aus Fig. 5 hervorgeht, die Form eines fünfzackigen Sterns aufweisen kann. Alternativ wäre auch vorstellbar, anstatt der aufgedampften Schicht 9 eine Folie, ein Plättchen oder ein Pulver zwischen den Körpern 3,4 anzuordnen. Die Schicht 9, die Folie, das Plättchen bzw. das Pulver sind in der bevorzugten Ausführungsform aus Gold gebildet.

Auf der Leuchtdiode 2 gegenüberliegenden Seite des Bauteils 16 kann ein Kontaktelement 8 aus Bergkristall angeordnet sein, das zur Auflage auf die Haut eines Patienten vorgesehen ist. Das Kontaktelement 8 weist eine zylindrische Form auf und ist an seinem auf den Patienten aufzusetzenden Ende mit einer konisch zusammenlaufenden Spitze versehen. Das Kontaktelement 8 weist in seinem zylindrischen Bereich einen derartigen Durchmesser auf, dass es in die Hülse 5 passt und dort verschiebbar ist. Um die Position des Kontaktelements 8 in der Hülse 5 festzustellen, ist in der Hülse 5 eine Gewindebohrung zur Aufnahme einer Schraube 14 vorgesehen, mit der sich das Kontaktelement in der Hülse 5 blockieren lässt.

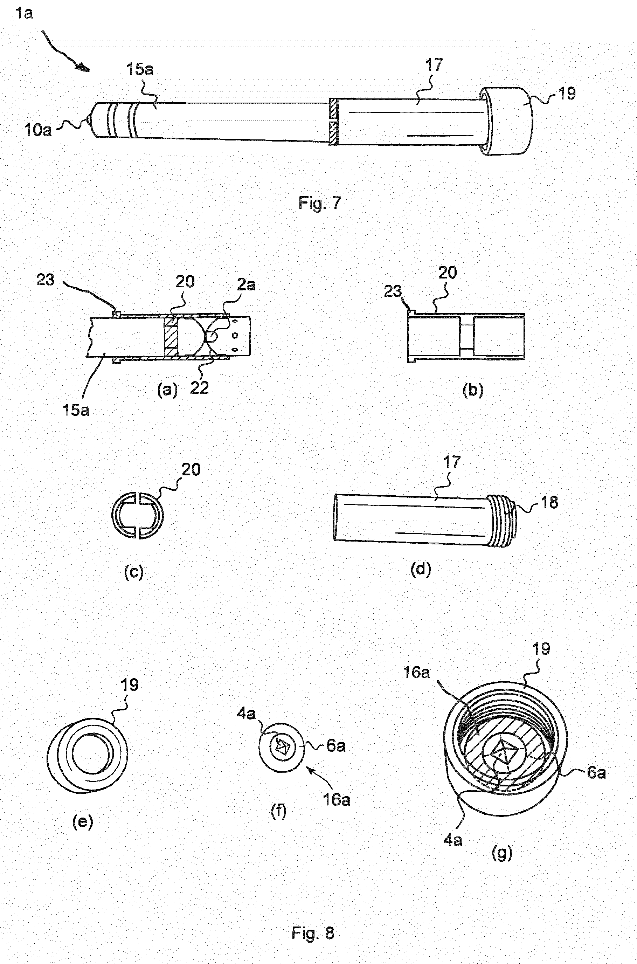

Es wird nun auf die Fig. 7 bis 9 Bezug genommen, wo gleiche oder gleichwirkende Teile mit derselben Bezugszahl wie in den Fig. 1 bis 6 bezeichnet sind und der betreffenden Bezugszahl jeweils der Buchstabe a beigefügt ist.

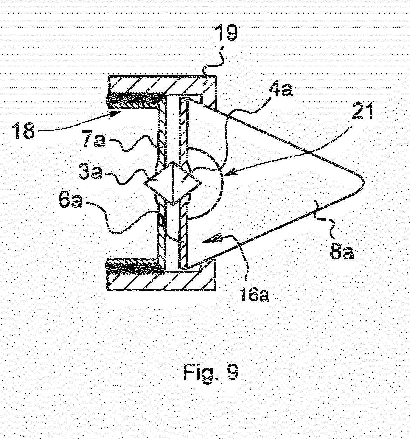

Das erfindungsgemäße Therapiegerät 1 a, das in Fig. 7 gezeigt ist, umfasst eine LED-Lampe mit einem Trägerelement 15a. Auf das Trägerelement 15a sind Halteelemente 20 aufgesetzt, auf die ein Silberrohr 17 aufgesteckt ist, das an seinem einen Ende mit einem Gewinde 18 versehen ist. Auf das Gewinde 18 ist ein Haltekörper 19 aufgeschraubt, der mit einem Innengewinde versehen ist, welches zum Eingreifen in das Gewinde 18 des Silberrohrs 17 vorgesehen ist. Wie insbesondere den Fig. 8e und 8g zu entnehmen ist, weist der Haltekörper 19 eine deckelartige Form auf und ist mit einer kreisrunden Ausnehmung versehen. Auf das Ende des Silberrohrs 17, an dem das Gewinde 18 vorgesehen ist, ist eine Baueinheit 16a einsetzbar, die im Wesentlichen so aufgebaut ist, wie es oben anhand der Fig. 2 für die Baueinheit 16 beschrieben ist. Abweichend von der Baueinheit 16 ist der Körper 3a auf seiner dem anderen Körper 4a zugewandten Seite mit einer hier nicht gezeigten Gravur versehen, die die Form von Außenkonturen eines fünfzackigen Sterns aufweist und mit Goldstaub gefüllt ist. Ferner können von der in Fig. 9 gezeigten Ausführungsform Scheiben 6a,7a, die die Körper 3a,4a zusammenhalten, direkt, beispielsweise mittels Punktschweißen, miteinander verbunden sein und ggf. zumindest abschnittsweise aneinanderliegen.

Wie insbesondere den Fig. 8 bis 10 zu entnehmen ist, ist das Halteelement 20 aus zwei Teilen gebildet, die sich von außen auf das Trägerelement 15a aufstecken lassen. Das Halteelement 20 weist dann einen derartigen Außendurchmesser auf, dass das Silberrohr 17 von dem Trägerelement 20 festgehalten wird. Als Anschlag für das Silberrohr 17 sind an dem Trägerelement an seinem einer Leuchtdiode 2a abgewandten Ende Auskragungen 23 vorgesehen.

Wie Fig. 9 zu entnehmen ist, kann zwischen das Ende des Silberrohrs 17 und dem Halteelement 19 neben der Baueinheit 16a ein konusförmiges Kontaktelement 8a eingeklemmt werden. Das konusartige Kontaktelement 8a ist auf seiner mit dem Bauelement 16a anzuordnenden Seite mit einer Auswölbung 21 versehen, in welche der Körper 4a vorsteht. Zur Befestigung der Baueinheit 16a und des Kontaktelements 8a werden diese mittels des Halteelements 19 gegen das Silberrohr 17 verklemmt, in dem das Halteelement 19 auf das Gewinde 18 aufgeschraubt wird.

Der Patient kann mit dem Therapiegerät 1 auf verschiedene Arten behandelt werden.

In einer ersten Behandlungsart ist in der Hülse 5 das Kontaktelement 8 nicht vorgesehen. Die Hülse 5 wird im Bereich von Chakren des Patienten auf die Haut aufgesetzt und mittels des Schalters 10 die LED 2 eingeschaltet. Von der LED 2 ausgestrahltes Kaltlicht tritt zunächst durch den Körper 4, ggf. dann durch die Folie 9 und anschließend durch den Körper 3 und von dort auf die Haut des Patienten.

In einer weiteren Behandlungsart ist in der Hülse 5 das Kontaktelement 8 vorgesehen. Zur Behandlung wird das Kontaktelement 8 mit der Spitze auf einen Akupunkturpunkt des Patienten aufgesetzt. Die Bestrahlung unterscheidet sich von der nach der ersten Behandlungsart beschriebenen Bestrahlung dadurch, dass das von der LED 2 ausgesendete Kaltlicht nach Austritt aus dem Körper 3 in das Kontaktelement 8 eintritt und aus der Spitze des Kontaktelements 8 auf die Haut trifft. Es versteht sich, dass mehrere Akupunkturpunkte nacheinander behandelt werden können oder mit mehreren Therapiegeräten 1 gleichzeitig mehrere Akupunkturpunkte behandelbar sind.

Durch die Behandlung mit dem Therapiegerät konnte chronisches Ekzem einer Patientin mit dem Behandlungserfolg erzielt werden, dass die von dem Ekzem verursachten Rötungen zunächst nachhaltig abblassten und nach mehrfacher Behandlung verschwanden.

Ferner ließen sich dauerhafte Kopf- und Rückenschmerzen lindern und beheben.

Die nachfolgenden Tabellen 1 und 2 zeigen Messungen an mehreren Patienten (Patienten Nr. 1 bis 7) vor und nach der Behandlung mittels des Therapiegeräts. Bei der Behandlung wurde zunächst eine Baueinheit 16 verwendet, bei der der Körper 4 aus schwarzem Opal und der Körper 3 aus Amethyst gebildet ist. Die Behandlung wurde am Sakralchakra durchgeführt. Das Kontaktelement 8 wurde nicht verwendet, d.h. die Strahlung wurde direkt auf die Haut des Patienten am Sakralchakra gerichtet.

In einem weiteren Behandlungsschritt wurde eine mit einem schwarzen Opal und einem Bergkristall besetzte Baueinheit 16 verwendet, wobei die Strahlung, ebenfalls ohne Verwendung des Kontaktelements 8, auf den Wurzelchakra gerichtet wurde. Die Messungen sind mit dem Messsystem "Global Diagnostics" der Vitatec AG (Vitalfeld-Analyse) durchgeführt worden.

Wie den Messungen zu entnehmen ist, konnten durch die Behandlung mit dem Therapiegerät deutliche Reaktionen der Patienten auf die Behandlung erzielt werden.

Weitere Behandlungserfolge an verschiedenen Patienten (Patienten Nr. 8 bis 22) sind in der nachfolgenden Tabelle 3 gezeigt. Zur Behandlung wurden mehrere der Therapiegeräte 1 a nach den Fig. 7 bis 9 benutzt, wobei in einer jeweils verwendeten Baueinheit 16a der Körper 4a aus schwarzem Opal und der Körper 3a aus den Materialien, die in der Tabelle 3 unter "Therapiegeräte bestückt mit" aufgeführt sind, besetzt waren. Die jeweiligen Therapiegeräte wurden gleichzeitig auf die in der Tabelle 3 genannten Behandlungsstellen am Körper gerichtet und die jeweiligen Behandlungsstellen gleichzeitig 10 Minuten lang bestrahlt.

Wie der Tabelle zu entnehmen ist, wurden zur Behandlung der Patienten unterschiedlich bestückte Therapiegeräte verwendet. Der Behandlungserfolg ist in der Tabelle in % in Abhängigkeit von der Anzahl von Tagen nach der ersten Behandlung aufgeführt.

**Tab. 1**

| Patient Nr.: | 1 | | 2 | | 3 | | 4 | | 5 | | 6 | | 7 | |
|---|---|---|---|---|---|---|---|---|---|---|---|---|---|---|
| vor/nach Behandlung | vor | nach | vor | nach | vor | nach | vor | nach | vor | nach | vor | nach | vor | nach |
| Verfügbare Energie | 69 % | 88 % | 68 % | 51 % | 77 % | 83 % | 72 % | 75 % | 87 % | 84 % | 80 % | 87 % | 60 % | 59 % |
| Hemmende Energie | 25 % | 8 % | 27 % | 48 % | 21 % | 10 % | 27% | 22 % | 8 % | 6 % | 19 % | 10 % | 31 % | 38 % |
| Degenerative Prozess | 33 % | 13 % | 49 % | 59 % | 38 % | 28 % | 40% | 50 % | 17 % | 37 % | 57 % | 37 % | 14 % | 34 % |
| Allgemeine Regulationsfähigkeit | 69 % | 66 % | 68 % | 17 % | 35 % | 57 % | 44% | 40 % | 77 % | 57 % | 59 % | 48 % | 57 % | 53 % |
| Schleimhäute | 21 % | 21 % | 14 % | 21 % | 29 % | 14 % | 21 % | 29 % | 38% | 21 % | 21 % | 14 % | 29 % | 14 % |
| Immunsystem | 15 % | 47 % | 15 % | 5 % | 28 % | 23 % | 53 % | 13 % | 15% | 18 % | 8 % | 23 % | 32 % | 23 % |
| aktive Toxine | 55 % | 26 % | 31 % | 42 % | 32 % | 43 % | 32 % | 25 % | 51 % | 22 % | 12 % | 20 % | 35 % | 35 % |
| Hormon Mimics | 37 % | 23 % | 23 % | 20 % | 20 % | 20 % | 23 % | 13 % | 47 % | 13 % | 7 % | 13 % | 20 % | 20 % |
| Metalle / Schwermetalle | 43 % | 23 % | 23 % | 50 % | 30 % | 40 % | 20 % | 20 % | 37 % | 13 % | 13 % | 13 % | 47 % | 33 % |
| Pharmaka / Geschmacksverstärker | 40 % | 10 % | 20 % | 20 % | 35 % | 33 % | 27 % | 20 % | 27% | 20 % | 7 % | 17% | 10 % | 23 % |
| Speicherbelastung | 40 % | 34 % | 43 % | 31 % | 35 % | 20 % | 37 % | 35 % | 31 % | 40 % | 37 % | 31 % | 40 % | 37 % |
| Filtersysteme | - | - | --- | - | --- | --- | - | - | 0 | 0 | -3 | -1 | -3 | -1 |
| Interstitium / Pischingerraum | 37 % | 33 % | 83 % | 20 % | 30 % | 23 % | 43 % | 30 % | 19 % | 30 % | 20 % | 23 % | 63 % | 43 % |
| Allergieneigung/ Reaktion | --- | - | --- | - | - | - | - | - | -1 | -1 | -1 | -1 | -3 | -1 |
| Muskulator | --- | --- | --- | --- | --- | --- | - | --- | -1 | -2 | -3 | -1 | -3 | -3 |
| Darm | -2 | -3 | -2 | -2 | -2 | -2 | -2 | -2 | -2 | -2 | -2 | 0 | 0 | 0 |
| Magen | -2 | 0 | 0 | -2 | 0 | -2 | -2 | 0 | -2 | 0 | 0 | -2 | 0 | 0 |
| Pankreas | 0 | -2 | 0 | -2 | -2 | -2 | 0 | 0 | -2 | 0 | -2 | 0 | 0 | 0 |
| Lunge | 0 | 0 | 0 | 0 | 0 | 0 | 0 | 0 | 0 | 0 | 0 | 0 | 0 | 0 |

**Tab. 2**

| Patient Nr.: | 1 | | 2 | | 3 | | 4 | | 5 | | 6 | | 7 | |
|---|---|---|---|---|---|---|---|---|---|---|---|---|---|---|
| Herz | -2 | 0 | 0 | 0 | -2 | 0 | 0 | 0 | 0 | 0 | 0 | 0 | 0 | 0 |
| Lymphe | -2 | 0 | -2 | -2 | 0 | 0 | -2 | -2 | -2 | -2 | 0 | 0 | 0 | -1 |
| Leber/Galle | -2 | 0 | 0 | 0 | 0 | -2 | 0 | -2 | 0 | 0 | -2 | 0 | 0 | 0 |
| Niere/Blase | 0 | 0 | -2 | 0 | 0 | -2 | 0 | 0 | 0 | 0 | -2 | 0 | 0 | 0 |
| Archaisches Immunsystem | 14 % | 3 % | 27 % | 3 % | 3 % | 20 % | 18 % | 3 % | 22 % | 3 % | 3 % | 28 % | 18 % | 16 % |
| Nitrosativer Stress | 29 % | 27 % | 31 % | 56 % | 27 % | 49 % | 38 % | 27 % | 49 % | 18 % | 22 % | 7 % | 33 % | 4 % |
| Oxydativer Stress | 40 % | 13 % | 47 % | 13 % | 27 % | 43 % | 60 % | 27 % | 40 % | 60 % | 33 % | 43 % | 43 % | 20 % |
| Glutathion | - | - | 0 | 0 | - | 0 | 0 | 0 | 0 | -1 | -1 | -1 | 0 | 0 |
| Hormon-Achse: | | | | | | | | | | | | | | |
| Hypothalamus | -1 | 0 | 0 | 0 | 0 | 0 | 0 | 0 | 0 | 0 | 0 | 0 | 0 | 0 |
| Hypophyse | 0 | -1 | -2 | 0 | 0 | 0 | 0 | 0 | 0 | 0 | 0 | 0 | 0 | 0 |
| Nebennieren | -1 | 0 | -1 | 0 | 0 | -1 | 0 | 0 | -1 | 0 | 0 | 0 | 0 | 0 |
| Oberbauch-Achse: | | | | | | | | | | | | | | |
| Leber | -1 | 0 | 0 | 0 | 0 | 0 | 0 | 0 | 0 | -1 | 0 | 0 | 0 | 0 |
| Magen | -1 | 0 | 0 | -1 | 0 | 0 | -1 | 0 | -1 | 0 | 0 | 0 | 0 | 0 |
| Pankreas | 0 | -1 | 0 | -1 | -1 | -1 | 0 | -1 | -1 | -1 | -1 | 0 | 0 | 0 |
| Milz | 0 | 0 | 0 | -1 | 0 | -2 | -1 | 0 | 0 | -1 | 0 | 0 | 0 | -1 |
| Stoffwechsel-Achse: | | | | | | | | | | | | | | |
| Schilddrüse | 0 | 0 | 0 | -1 | 0 | 0 | -1 | 0 | 0 | 0 | 0 | 0 | 0 | 0 |
| Pankreas | 0 | 0 | 0 | 0 | 0 | 0 | 0 | 0 | 0 | 0 | 0 | 0 | 0 | 0 |
| Magen | -1 | 0 | 0 | -1 | -1 | -1 | -1 | -1 | -1 | -1 | 0 | -1 | -1 | -1 |
| Dünndarm | -1 | -2 | -2 | -1 | -1 | -1 | -1 | -1 | -2 | -1 | -1 | -1 | -1 | -1 |
| Dickdarm | -1 | 0 | 0 | 0 | 0 | 0 | -1 | -1 | -1 | -1 | 0 | 0 | 0 | 0 |
| Wirbelsäulen-Achse: | | | | | | | | | | | | | | |
| Steuerungsebene | -2 | -1 | -1 | -1 | -1 | 0 | -1 | -1 | -1 | -1 | 0 | 0 | -1 | 0 |
| Kiefer | 0 | -1 | -1 | -1 | -1 | 0 | -1 | 0 | 0 | -1 | 0 | 0 | -1 | 0 |
| Zwerchfell | -1 | 0 | 0 | -1 | -1 | 0 | 0 | 0 | -1 | 0 | 0 | 0 | 0 | 0 |
| Beckenboden | -1 | 0 | -1 | 0 | -2 | -1 | 0 | -1 | -2 | -1 | -1 | -1 | 0 | -2 |

**Tab. 3**

| Patient Nr.: | Diagnose / Beschwerden: | Behandlung Nr.: | Datum der Behandlung: | Therapiegeräte bestückt mit: | Behandlungsstelle am Körper: | Besserung der Beschwerden in % nach Anzahl [Tage] nach Erstbehandlung: |
|---|---|---|---|---|---|---|
| 8 | Depressionen mit Müdigkeit und Nervosität | 1 | 23.4.2013 | • Blauer Saphir | • Hals-Chakra | 70 % [1] |
| | | | | • Rosa Turmalin | • Herz-Chakra | |
| | | | | • Wassermelonenturmalin | • zw. Hals- und Herz-Chakra | |
| | | 2 | 7.5.2013 | • Diamant | • Wurzel-Chakra | 100 % [14] |
| | | | | • Amethyst | • Sakral-Chakra | |
| 9 | Schwere Depression seit 10 Jahren | 1 | 4.6.2013 | • Smaragd | • Stirn-Chakra | 50 % [3] |
| | | | | • Aquamarin | • zw. Hals- und Stirn-Chakra | |
| | | | | • Blauer Saphir | • Hals-Chakra | |
| | | 2 | 11.6.2013 | • Diamant | • Wurzel-Chakra | 80 % [7] |
| | | | | • Amethyst | • Sakral-Chakra | |
| 10 | COPD (chronic obstrucive pulmonary disease) Stadium 4 (bisher keine medizinische Therapiemöglichkeit), schwere Depression | 1 | 9.7.2013 | • Blauer Saphir | • Hals-Chakra | |
| | | | | • Rosa Turmalin | • Herz-Chakra | |
| | | | | • Wassermelonenturmalin | • zw. Hals- und Herz-Chakra | |
| | | 2 | 12.7.2013 | • Blauer Saphir | • Hals-Chakra | 30 % [3] |
| | | | | • Rosa Turmalin | • Herz-Chakra | |
| | | | | • Wassermelonenturmalin | • zw. Hals- und Herz-Chakra | |
| | | 3 | 7.8.2013 | • Blauer Saphir | • Hals-Chakra | 50-60 % [29] |
| | | | | • Rosa Turmalin | • Herz-Chakra | |
| | | | | • Wassermelonenturmalin | • bzw. Hals- und Herz-Chakra | |
| | | 4 | 14.8.2013 | • Rubin | • Solarplexus | 40 % [36] |
| | | | | • Diamant | • Wurzel-Chakra | |
| | | | | • Amethyst | • Sakral-Chakra | |
| | | 5 | 22.8.2013 | • Rubin | • Solarplexus | 70 % [43] |
| | | | | • Diamant | • Wurzel-Chakra | |
| | | | | • Amethyst | • Sakral-Chakra | |
| 11 | Insulinpfl. Diabetes, Übelkeit, epigastische Schmerzen, Parästhesien; Angste | 1 | 9.4.2013 | • Rubin | • Solarplexus | |
| | | | | • Diamant | • Wurzel-Chakra | |
| | | | | • Amethyst | • Sakral-Chakra | |
| | | 2 | 14.5.2013 | • Rubin | • Solarplexus | 70 % [35] |
| | | | | • Diamant | • Wurzel-Chakra | |
| | | | | • Amethyst | • Sakral-Chakra | |
| | | 3 | 13.6.2013 | • Rubin | • Solarplexus | 90 % [65] |
| | | | | • Diamant | • Wurzel-Chakra | |
| | | | | • Amethyst | • Sakral-Chakra | |
| | | 4 | 6.8.2013 | • Rubin | • Solarplexus | 100% [119] |
| | | | | • Diamant | • Wurzel-Chakra | |
| | | | | • Amethyst | • Sakral-Chakra | |
| 12 | Massives Ekzem mit erheblicher Rötung im Gesicht, Hals und am Oberkörper | 1 | 27.3.2013 | • Amethyst | • Sakral-Chakra | 50 % [3] |
| | | | | • Diamant | • Wurzel-Chakra | |
| | | | | • Rubin | • Solarplexus | |
| | | | | • Rosa Turmalin | • Herz-Chakra | |
| | | 2 | 22.4.2013 | • Smaragd | • Stirn-Chakra | 80 % [28] |
| | | | | • Blauer Saphir | • Hals-Chakra | |
| 13 | therapieresistente Schmerzen, UB, Depressionen, Ängste | 1 | 16.4.2013 | • Diamant | • Wurzel-Chakra | |
| | | | | • Amethyst | • Sakral-Chakra | |
| | | 2 | 24.4.2013 | • Blauer Saphir | • Hals-Chakra | |
| | | | | • Rosa Turmalin | • Herz-Chakra | |
| | | | | • Wassermelonenturmalin | • zw. Hals- und Herz-Chakra | |
| | | 3 | 2.5.2013 | • Diamant | • Wurzel-Chakra | 100% [16] |
| | | | | • Amethyst | • Sakral-Chakra | |
| | | 4 | 28.5.2013 | • Diamant | • Wurzel-Chakra | 70 % [42] |
| | | | | • Amethyst | • Sakral-Chakra | |
| | | 5 | 2.7.2013 | • Rubin | • Solarplexus | 70 % [77] |
| | | | | • Diamant | • Wurzel-Chakra | |
| | | | | • Amethyst | • Sakral-Chakra | |
| | | 6 | 10.7.2013 | • Rubin | • Solarplexus | 80 % [86] |
| | | | | • Diamant | • Wurzel-Chakra | |
| | | | | • Amethyst | • Sakral-Chakra | |
| 14 | Parasternaler Druck Tachycardie, Fehlwahrnehmungen | 1 | 18.6.2013 | • Blauer Saphir | • Hals-Chakra | |
| | | | | • Rosa Turmalin | • Herz-Chakra | |
| | | | | • Aquamarin | • zw. Hals- und Stirn-Chakra | |
| | | 2 | 9.7.2013 | • Blauer Saphir | • Hals-Chakra | 100% [21] |
| | | | | • Rosa Turmalin | • Herz-Chakra | |
| | | | | • Wassermelonenturmalin | • zw. Hals- und Herz-Chakra | |
| 15 | 1: genital. Herpes | 1 | 11.4.2013 | • Blauer Saphir | • Hals-Chakra | |
| | 2: Dermatitis Gesicht | | | • Aquamarin | • zw. Hals- und Stirn-Chakra | |
| | | | | • Smaragd | • Stirn-Chakra | |
| | | 2 | 17.4.2013 | • Diamant | • Wurzel-Chakra | 1: 50% [7] |
| | | | | • Amethyst | • Sakral-Chakra | 2: 100 % [7] |
| 16 | Schwerstes generalisiertes Ekzem ganzer Körper seit 10 Jahren (keine Therapiemöglichkeit) | 1 | 22.3.2013 | • Diamant | • Wurzel-Chakra | |
| | | | | • Amethyst | • Sakral-Chakra | |
| | | 2 | 22.4.2013 | • Herkimer Diamant | • Scheitel-Chakra | 60% [31] |
| | | | | • Smaragd | • Stirn-Chakra | |
| | | 3 | 6.5.2013 | • Blauer Saphir | • Hals-Chakra | 80 % [45] |
| | | | | • Wassermelonenturmalin | • zw. Hals- und Herz-Chakra | |
| | | 4 | 24.6.2013 | • Blauer Saphir | • Hals-Chakra | 90 % [94] |
| | | | | • Smaragd | • Stirn-Chakra | |
| | | | | • Aquamarin | • zw. Hals- und Stirn-Chakra | 100% [112] |
| 17 | MCS, CFS, | 1 | 10.4.2013 | • Diamant | • Wurzel-Chakra | 70 % [2] |
| | Polyneuropath. morphinbedürftige Schmerzen | | | • Amethyst | • Sakral-Chakra | |
| 18 | Anaplastischer Hirntumor seit 12 Jahren | 1 | 3.7.2013 | • Herkimer Diamant | • Scheitel-Chakra | |
| | 1: Schwäche | | | • Smaragd | • Stirn-Chakra | |
| | 2: Blässe | | | • Amethyst | • Sakral-Chakra | |
| | 3: Bauchschmerzen | | | | | |
| | 4: Kopfschmerzen | | | | | |
| | | 2 | 10.7.2013 | • Blauer Saphir | • Hals-Chakra | 1: 60 % [7] |
| | | | | • Diamant | • Wurzel-Chakra | 2: 60 % [7] |
| | | | | • Amethyst | • Sakral-Chakra | 3: 80 % [7] |
| | | | | | | 4: 80 % [7] |
| | | 3 | 9.8.2013 | • Blauer Saphir | • Hals-Chakra | 1: 80 % [38] |
| | | | | • Herkimer Diamant | • Scheitel-Chakra | 2: 100 % [38] |
| | | | | | | 3: 80 % [38] |
| | | | | • Diamant | • Wurzel-Chakra | 4: 90 % [38] |
| | | | | • Amethyst | • Sakral-Chakra | |
| 19 | Übelkeit, Schwindel, Tachycardie | 1 | 20.8.2013 | • Rubin | • Solarplexus | 100% [1] |
| | | | | • Amethyst | • Sakral-Chakra | |
| | | | | • Diamant | • Wurzel-Chakra | |
| 20 | 1: therapieresistenter Schwindel und Übelkeit | 1 | 22.4.2013 | • Blauer Saphir | • Hals-Chakra | |
| | | | | • Smaragd | • Stirn-Chakra | |
| | 2: massive Gastritis (stat) | | | | | |
| | | 2 | 30.4.2013 | • Rubin | • Solarplexus | 60 % [8] |
| | | | | • Wassermelonenturmalin | • zw. Hals- und Herz-Chakra | |
| | | 3 | 3.5.2013 | • Amethyst | • Sakral-Chakra | |
| | | | | • Diamant | • Wurzel-Chakra | |
| | | 4 | 4.6.2013 | • Rubin | • Solarplexus | 1: 60 % [43] |
| | | | | • Diamant | • Wurzel-Chakra | 2: 100 % [43] |
| | | | | • Amethyst | • Sakral-Chakra | |
| 21 | CFS, MCS; chronische Dysbiose, häufige Virusinfekte | 1 | 16.4.2013 | • Rosa Turmalin | • Herz-Chakra | 20 % [3] |
| | | | | • Wassermelonenturmalin | • zw. Hals- und Herz-Chakra | |
| | | 2 | 29.4.2013 | • Diamant | • Wurzel-Chakra | 70% [13] |
| | | | | • Amethyst | • Sakral-Chakra | |
| 22 | Ängste, große Schwäche | 1 | 3.7.2013 | • Blauer Saphir | • Hals-Chakra | |
| | | | | • Rubin | • Solarplexus | |
| | | | | • Amethyst | • Sakral-Chakra | |
| | | 2 | 5.7.2013 | • Blauer Saphir | • Hals-Chakra | 30-50% [2] |
| | | | | • Rubin | • Solarplexus | |
| | | | | • Amethyst | • Sakral-Chakra | |
| | | | | • Smaragd | • Stirn-Chakra | |
| | | 3 | 10.7.2013 | • Blauer Saphir | • Hals-Chakra | 40-60 % [7] |
| | | | | • Diamant | • Wurzel-Chakra | |
| | | | | • Amethyst | • Sakral-Chakra | |

## Patentansprüche

1. Therapiegerät, umfassend eine Strahlungsquelle (2), insbesondere eine Lichtquelle, zur Bestrahlung eines Patienten, wobei
das Therapiegerät (1) zumindest zwei Körper (3,4) aufweist, die sich durch das jeweilige Material, durch das sie gebildet sind, voneinander unterscheiden, die mit ihren Grundflächen (11,12) unmittelbar oder über eine Zwischenlage (9) aneinander anliegen und im Strahlengang des Therapiegerätes (1) hintereinander angeordnet sind,
**gekennzeichnet dadurch, dass**:
einer der Körper (3) aus Opal und der andere oder die anderen Körper (4) aus Kristall oder aus einem Materialgerüst einer Koralle gebildet ist bzw. sind
und dass die zumindest zwei Körper (3,4) pyramidenartig geformt sind.

2. Therapiegerät nach Anspruch 1,
**dadurch gekennzeichnet,**
**dass** einer der Körper (3), vorzugsweise der im Strahlengang vordere Körper (3), aus schwarzem Opal und der andere Körper (4), vorzugsweise der im Strahlengang hintere Körper (4), aus Bergkristall, Saphir, Herkimer Diamant, rosa Turmalin, Diamant, Smaragd, Rubin, Aquamarin, rosa Koralle, Wassermelonenturmalin oder Amethyst gebildet ist.

3. Therapiegerät nach Anspruch 1 oder 2,
**dadurch gekennzeichnet,**
**dass** die Zwischenlage (9) durch eine aufgedampfte dünne Schicht (9), ein Plättchen, eine Folie und/oder durch ein Pulver gebildet ist, wobei die Zwischenlage (9) vorzugsweise aus Gold gebildet ist.

4. Therapiegerät nach einem der Ansprüche 1 bis 3,
**dadurch gekennzeichnet,**
**dass** zumindest einer der Körper (3,4) an der Fläche, mit der er an dem anderen Körper (3,4) anliegt, mit einer Gravur versehen ist, die vorzugsweise die Form von Außenkonturen eines Sterns, bevorzugt eines 5-zackigen Sterns, aufweist.

5. Therapiegerät nach Anspruch 4,
**dadurch gekennzeichnet,**
**dass** in der Gravur ein Pulver, vorzugsweise Goldpulver, angeordnet ist.

6. Therapiegerät nach einem der Ansprüche 1 bis 5,
**dadurch gekennzeichnet,**
**dass** die Körper (3,4) durch zwei, vorzugsweise aus Silber gebildeten und mit einer Ausnehmung zur Aufnahme der Körper versehenen, Scheiben (6,7) zusammengehalten sind.

7. Therapiegerät nach einem der Ansprüche 1 bis 6,
**dadurch gekennzeichnet,**
**dass** im Strahlengang hinter den Körpern (3,4) ein Kontaktelement (8) zur Anlage an dem Patienten vorgesehen ist.

8. Therapiegerät nach Anspruch 7,
**dadurch gekennzeichnet,**
**dass** das Kontaktelement (8) aus Bergkristall besteht.

9. Therapiegerät nach Anspruch 7 oder 8,
**dadurch gekennzeichnet,**
**dass** das Kontaktelement (8) eine zylindrische Form aufweist und sich vorzugsweise an dem Ende, mit dem es an den Patienten anzulegen ist, konusartig verjüngt.

10. Therapiegerät nach Anspruch 7 oder 8,
**dadurch gekennzeichnet,**
**dass** das Kontaktelement (8a) konusartig geformt ist und auf seiner Strahlungsquelle (2a) zugewandten Seite mit einer Auswölbung (21) versehen ist, in welche ein Teil des im Strahlengang hinteren Körpers (4a) vorstehen kann.

11. Therapiegerät nach einem der Ansprüche 1 bis 10,
**gekennzeichnet durch** eine rohrförmige, vorzugsweise aus Silber gebildete, Hülse (5;17) zur Aufnahme der Strahlungsquelle (2), der Körper (3,4) und ggf. des Kontaktelements (8).

12. Therapiegerät nach einem der Ansprüche 8 bis 11,
**dadurch gekennzeichnet,**
**dass** der Abstand zwischen der Strahlungsquelle (2) und den Körpern (3,4) verstellbar ist.

13. Therapiegerät nach einem der Ansprüche 8 bis 12,
**dadurch gekennzeichnet,**
**dass** die Position des Kontaktelements (8) in der Hülse (5) verstellbar ist.

14. Therapiegerät nach einem der Ansprüche 1 bis 13,
**dadurch gekennzeichnet,**
**dass** die Strahlungsquelle (2) zum Strahlen im Wellenlängenbereich des sichtbaren Lichts, vorzugsweise im Wellenlängenbereich von 460 bis 480 nm, vorgesehen ist.

## Claims

1. Therapy device comprising a radiation source (2), in particular a light source, for irradiating a patient, wherein the therapy device (1) has at least two bodies (3, 4), which differ from each other in terms of the respective material by which they are formed and which bear with their base surfaces (11, 12) directly on each other or via an intermediate layer (9) and are arranged one behind the other in the beam path of the therapy device (1), **characterized in that** one of the bodies (3) is formed from opal and the one or more other bodies (4) from crystal or from a material skeleton of a coral, and **in that** the at least two bodies (3, 4) are pyramid-shaped.

2. Therapy device according to Claim 1, **characterized in that** one of the bodies (3), preferably the front body (3) in the beam path, is formed from black opal, and the other body (4), preferably the rear body (4) in the beam path, is formed from rock crystal, sapphire, Herkimer diamond, pink tourmaline, diamond, emerald, ruby, aquamarine, pink coral, watermelon tourmaline or amethyst.

3. Therapy device according to Claim 1 or 2, **characterized in that** the intermediate layer (9) is formed by a vapour-deposited thin coat (9), a wafer, a foil and/or by a powder, wherein the intermediate layer (9) is preferably formed from gold.

4. Therapy device according to one of Claims 1 to 3, **characterized in that** at least one of the bodies (3, 4) is provided, on the surface bearing on the other body (3, 4), with an engraving, which preferably has the form of outer contours of a star, preferably a five-pointed star.

5. Therapy device according to Claim 4, **characterized in that** a powder, preferably gold powder, is arranged in the engraving.

6. Therapy device according to one of Claims 1 to 5, **characterized in that** the bodies (3, 4) are held together by two discs (6, 7), which are preferably formed from silver and are provided with a recess for receiving the bodies.

7. Therapy device according to one of Claims 1 to 6, **characterized in that** a contact element (8) for placing on the patient is provided in the beam path behind the bodies (3, 4).

8. Therapy device according to Claim 7, **characterized in that** the contact element (8) is made of rock crystal.

9. Therapy device according to Claim 7 or 8, **characterized in that** the contact element (8) has a cylindrical shape and tapers like a cone, preferably at the end with which it is to be placed on the patient.

10. Therapy device according to Claim 7 or 8, **characterized in that** the contact element (8a) is shaped like a cone and, on its side facing towards the beam source (2a), is provided with a protuberance (21) into which a part of the rear body (4a) in the beam path can protrude.

11. Therapy device according to one of Claims 1 to 10, **characterized by** a tubular sleeve (5, 17), preferably formed from silver, for receiving the beam source (2), the bodies (3, 4) and, if appropriate, the contact element (8).

12. Therapy device according to one of Claims 8 to 11, **characterized in that** the distance between the radiation source (2) and the bodies (3, 4) is adjustable.

13. Therapy device according to one of Claims 8 to 12, **characterized in that** the position of the contact element (8) in the sleeve (5) is adjustable.

14. Therapy device according to one of Claims 1 to 13, **characterized in that** the radiation source (2) is provided for radiation in the wavelength range of visible light, preferably in the wavelength range from 460 nm to 480 nm.

## Revendications

1. Appareil de thérapie, comprenant une source de rayonnement (2), en particulier une source lumineuse, pour irradier un patient, l'appareil de thérapie (1) présentant au moins deux corps (3, 4) qui se distinguent l'un de l'autre par le matériau par lequel ils sont formés, qui sont placés l'un contre l'autre avec leur surface de base (11,12), directement ou via une couche intermédiaire (9), et qui sont placés l'un derrière l'autre dans le trajet des rayons de l'appareil de thérapie (1), **caractérisé en ce qu'**un des corps (3) est réalisé en opale et l'autre ou les autres corps (4) est/sont réalisés en cristal ou en une structure de matériau de corail et **en ce qu'**au moins deux corps (3, 4) présentent une forme de type pyramide.

2. Appareil de thérapie selon la revendication 1, **caractérisé en ce qu'**un des corps (3), de préférence le corps (3) antérieur dans le trajet de rayons, est réalisé en opale noire et l'autre corps (4), de préférence le corps (4) postérieur dans le trajet des rayons, est réalisé en cristal de roche, en saphir, en diamant de Herkimer, en tourmaline rose, en diamant, en émeraude, en rubis, en aigue-marine, en corail rose, en tourmaline melon d'eau ou en améthyste.

3. Appareil de thérapie selon la revendication 1 ou 2, **caractérisé en ce que** la couche intermédiaire (9) est formée par une couche mince (9) déposée par vaporisation, une plaquette, une feuille et/ou une poudre, la couche intermédiaire (9) étant de préférence réalisée en or.

4. Appareil de thérapie selon l'une quelconque des revendications 1 à 3, **caractérisé en ce qu'**au moins un des corps (3, 4) est pourvu, au niveau de la surface avec laquelle il se place contre l'autre corps (3, 4), d'une gravure qui présente de préférence la forme des contours externes d'une étoile, de préférence d'une étoile à 5 branches.

5. Appareil de thérapie selon la revendication 4, **caractérisé en ce qu'**une poudre, de préférence une poudre d'or, est disposée dans la gravure.

6. Appareil de thérapie selon l'une quelconque des revendications 1 à 5, **caractérisé en ce que** les corps (3, 4) sont maintenus ensemble par deux disques (6, 7), de préférence réalisés en argent et pourvus d'un évidement pour recevoir les corps.

7. Appareil de thérapie selon l'une quelconque des revendications 1 à 6, **caractérisé en ce qu'**un élément de contact (8), destiné à être placé sur le patient, est disposé dans le trajet de rayons, derrière les corps (3, 4).

8. Appareil de thérapie selon la revendication 7, **caractérisé en ce que** l'élément de contact (8) est constitué de cristal de roche

9. Appareil de thérapie selon la revendication 7 ou 8, **caractérisé en ce que** l'élément de contact (8) présente une forme cylindrique et se rétrécit en forme de cône, de préférence au niveau de l'extrémité avec laquelle il se place sur le patient

10. Appareil de thérapie selon la revendication 7 ou 8, **caractérisé en ce que** l'élément de contact (8a) est en forme de cône et est pourvu, sur sa face orientée vers la source de rayonnement (2a), d'une courbure (21) dans laquelle une partie du corps arrière (4a) dans le trajet des rayons peut être en saillie.

11. Appareil de thérapie selon l'une quelconque des revendications 1 à 10, **caractérisé par** une gaine (5 ; 17) tubulaire, de préférence réalisée en argent, destinée à recevoir la source de rayonnement (2), les corps (3, 4) et le cas échéant l'élément de contact (8).

12. Appareil de thérapie selon l'une quelconque des revendications 8 à 11, **caractérisé en ce que** la distance entre la source de rayonnement (2) et les corps (3, 4) est réglable.

13. Appareil de thérapie selon l'une quelconque des revendications 8 à 12, **caractérisé en ce que** la position de l'élément de contact (8) dans la gaine (5) est réglable.

14. Appareil de thérapie selon l'une quelconque des revendications 1 à 13, **caractérisé en ce que** la source de rayonnement (2) est destinée à irradier dans la plage de longueurs d'onde de la lumière visible, de préférence dans la plage de longueurs d'onde de 460 à 480 nm.
